# NEUE EUROPÄISCHE PATENTSCHRIFT

(11) **EP 2 675 403 B2**
(45) Veröffentlichungstag und Bekanntmachung des Hinweises auf die Entscheidung über den Einspruch: **12.03.2025**
(45) Hinweis auf die Patenterteilung: 07.12.2016
(21) Anmeldenummer: 12703793.5
(22) Anmeldetag: 10.02.2012
(51) Int. Cl.: A61F 2/86, A61F 2/90

(54) **MEDIZINISCHE VORRICHTUNG MIT EINEM EXPANDIERBAREN GITTERGEFLECHT**
MEDICAL DEVICE COMPRISING AN EXPANDABLE LATTICE MESH
DISPOSITIF MÉDICAL COMPRENANT UN TREILLIS MÉTALLIQUE EXPANSIBLE

(30) Priorität: 14.02.2011 DE 102011011180
(43) Veröffentlichungstag der Anmeldung: 25.12.2013
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Meissner Bolte Partnerschaft mbB
(86) Internationale Anmeldenummer: PCT/EP2012/052287
(87) Internationale Veröffentlichungsnummer: WO 2012/110414

(56) Entgegenhaltungen:
- EP-A2- 0 923 912
- WO-A1-01/35864
- WO-A1-01/54621
- WO-A1-2004/105647
- WO-A1-2008/130530
- WO-A1-2010/034453
- WO-A1-2012/082440
- DE-A1- 10 127 602
- DE-A1- 102009 006 180
- DE-A1- 19 750 971
- DE-U1- 202008 014 828
- FR-A1- 2 858 208
- GB-A- 2 470 484
- JP-A- 2006 296 559
- US-A1- 2002 179 166
- US-A1- 2003 149 475
- US-A1- 2004 153 117
- US-A1- 2005 137 692
- US-A1- 2007 208 373
- US-A1- 2008 275 540
- US-A1- 2009 054 972
- US-A1- 2009 082 840
- US-A1- 2009 177 262
- US-B1- 6 622 604

## Beschreibung

Die Erfindung betrifft eine medizinische Vorrichtung gemäß dem Oberbegriff des Patentanspruchs 1. Eine derartige medizinische Vorrichtung offenbart beispielsweise WO 2008/130530 A1.

Aus der WO 2008/130530 A1 ist ein Stent bekannt, der aus mehreren, miteinander verflochtenen bzw. sich überkreuzenden Drähten gebildet ist. Insbesondere bilden die sich mehrfach kreuzenden Drähte ein Gittergeflecht, das im Wesentlichen in einer zylinderförmigen Wandung des Stents angeordnet ist. Das Gittergeflecht bzw. allgemein der Stent ist expandierbar. Das bedeutet, dass der Stent von einem radial komprimierten Zustand, in dem der Stent einen relativ kleineren Querschnittsdurchmesser aufweist, in einem radial expandierten Zustand überführbar ist, in dem der Stent einen relativ größeren Querschnittsdurchmesser aufweist.

Bei dem bekannten Stent sind die Drähte, die das Gittergeflecht bilden, an einem Längsende des Stents umgelenkt und in das Gittergeflecht zurückgeführt. Auf diese Weise sind an dem Längsende des Stents Schlaufen gebildet. Dadurch werden an dem Längsende offene Drahtenden vermieden, so dass das Längsende atraumatisch wirksam ist.

In vielen Fällen ist es gewünscht, dass Stents im Allgemeinen eine hohe Feinmaschigkeit, also ein kleine Maschengröße bzw. Zellengröße aufweisen. Gleichzeitig ist eine ausreichende Radialkraft erforderlich, um den Stent sicher in einem Blutgefäß zu positionieren. Die Radialkraft des Stents ist jedoch entlang des Gittergeflechts nicht konstant. Vielmehr ändert sich die Radialkraft eines Stents von der Stentmitte zum Längsende des Stents hin. Dies erschwert die Herstellung eines Stents, der eine erhöhte Feinmaschigkeit aufweisen soll.

Die erhöhte Feinmaschigkeit wird durch die Verwendung relativ dünner Drähte erreicht, die allerdings eine vergleichsweise geringe Radialkraft bewirken. Durch die Verwendung von relativ dickeren Drähten, die eine erhöhte Radialkraft bewirken, wird die Feinmaschigkeit hingegen reduziert. Überdies wird bei dem Einsatz relativ dickerer Drähte für die Gitterstruktur der Querschnittsdurchmesser des Stents im komprimierten Zustand erhöht, so dass die Zufuhr des Stents durch einen Katheter erschwert ist. Dies gilt auch im Hinblick auf die Radialkraft, die bei einem Stent mit relativ dickeren Drähten im komprimierten Zustand auf die Katheterinnenwand wirkt und somit erhöhte Reibungskräfte verursacht, die das Zuführen des Stents erschweren.

Der Unterschied zwischen der Radialkraft, die der bekannte Stent an seinem Längsende aufweist, und der Radialkraft in der Mitte des Gittergeflechts, führt zu dem Nachteil, dass zwischen einer gewünschten Feinmaschigkeit und einer ausreichenden Radialkraft über die gesamte Länge des Gittergeflechts ein Kompromiss eingegangen werden muss. Aus den Druckschriften US 2009/0054972 A1, US 2003/0149475 ,A1, US 2009/0177262 A1, FR 2858204 A1 und US 6,622,604 B1 sind Stents mit Endschlaufen bekannt, wobei die Stents aus US 2003/0149475 A1 und FR 2858204 A1 jeweils eine Endschlaufe zeigen, die aus einem verstärkenden Draht gebildet sind. Diese einzelne Verstärkungsschlaufe beeinflusst jedoch in unerwünschter Weise das Expansionsverhalten des Stents und somit dessen Positionierbarkeit.

US 2005/0137692 A1 offenbart eine Herzklappenprothese mit einer Gitterstruktur, die aus mehreren überkreuzten Drähten gebildet ist. Die Drähte können unterschiedliche Drahtstärken aufweisen. Im Vordergrund steht bei der Herzklappenprothese eine ausreichende Radialkraft der Gitterstruktur, die hohen mechanischen Belastungen standhalten muss.

Die Aufgabe der Erfindung besteht darin, eine medizinische Vorrichtung anzugeben, die einfach handhabbar ist, wobei eine Radialkraft zumindest bereichsweise weitgehend unabhängig von der gewünschten Feinmaschigkeit einstellbar ist.

Erfindungsgemäß wird diese Aufgabe durch den Gegenstand des Patentanspruchs 1 gelöst.

Die Erfindung beruht auf dem Gedanken, eine medizinische Vorrichtung mit einem expandierbaren Gittergeflecht aus sich überkreuzenden Drähten anzugeben, die an wenigstens einem Längsende des Gittergeflechts Schlaufen bilden, wobei wenigstens eine Schlaufe als Verstärkungsschlaufe ausgebildet ist. Die Verstärkungsschlaufe ist durch einen Draht gebildet, der im Bereich der Verstärkungsschlaufe einen größeren Querschnittsdurchmesser als die Drähte aufweist, die die anderen Schlaufen bilden.

Die erfindungsgemäße medizinische Vorrichtung hat den Vorteil, dass dem Längsende des Gittergeflechts durch die Verstärkungsschlaufe eine erhöhte Stabilität verliehen wird. Somit wird die Positionierung der medizinischen Vorrichtung innerhalb eines Körperhohlorgans verbessert. Konkret wird durch die Verstärkungsschlaufe eine Dislokation, also eine Verschiebung, der medizinischen Vorrichtung innerhalb des Körperhohlorgans vermieden.

Ein weiterer Vorteil der Erfindung besteht darin, dass zur Bildung der anderen Schlaufen Drähte eingesetzt werden können, die einen relativ kleinen Querschnittsdurchmesser aufweisen, so dass die Feinmaschigkeit des Gittergeflechts insgesamt erhöht wird. Die Radialkraft, die zu einer sicheren Positionierung der medizinischen Vorrichtung in ein Körperhohlorgan zweckmäßig ist, wird zumindest im Bereich des Längsendes durch den Draht der Verstärkungsschlaufe bereitgestellt. Auf diese Weise wird die Herstellung einer medizinischen Vorrichtung ermöglicht, die einerseits eine hohe Feinmaschigkeit und gleichzeitig eine ausreichend hohe Radialkraft aufweist. Dabei ist die Höhe der Radialkraft durch die Drahtstärke einzelner Drähte, die die Verstärkungsschlaufen bilden, einstellbar. Somit kann die Radialkraft zumindest am Längsende des Gittergeflechts weitgehend ohne Einfluss auf die Feinmaschigkeit des Gittergeflechts eingestellt werden.

Erfindungsgemäß sind mehrere Verstärkungsschlaufen vorgesehen, die musterartig über das Längsende verteilt angeordnet sind. Beispielsweise kann jede zweite Schlaufe oder jede dritte Schlaufe als Verstärkungsschlaufe ausgebildet sein. Andere Muster sind möglich. Auf diese Weise wird eine gleichmäßige Verteilung der Verstärkungsschlaufen über das Längsende erreicht, wodurch eine zentrierte Anordnung der medizinischen Vorrichtung in einem Körperhohlorgan erleichtert bzw. ein selbstzentrierendes Gittergeflecht bereitgestellt wird. Insgesamt wird dadurch die Positionierung der medizinischen Vorrichtung in einem Körperhohlorgan verbessert.

Bei der Erfindung sind höchstens 34%, insbesondere höchstens 17%, der Schlaufen als Verstärkungsschlaufen ausgebildet. Dies gewährleistet ein besonders vorteilhaftes Verhältnis zwischen Drähten des Gittergeflechts, die eine Erhöhung der Feinmaschigkeit bewirken und Drähten des Gittergeflechts, die zumindest im Bereich der Verstärkungsschlaufen eine erhöhte Radialkraft bereitstellen.

Die Verstärkungsschlaufen können in Längsrichtung des Gittergeflechts über die anderen Schlaufen vorstehen oder bezogen auf die anderen Schlaufen zurückgesetzt sein. Insgesamt ist ein Versatz zwischen den Verstärkungsschlaufen und den anderen Schlaufen vorteilhaft, da auf diese Weise eine Überlappung von Verstärkungsschlaufen mit anderen Schlaufen im komprimierten Zustand des Gittergeflechts reduziert ist. Der Querschnittsdurchmesser des Längsendes des Gittergeflechts im komprimierten Zustand wird auf diese Weise verringert. Das ermöglicht die Verwendung kleinerer Zuführsysteme bzw. die Anordnung der medizinischen Vorrichtung in relativ kleinen Körperhohlorganen.

Bei einer weiteren bevorzugten Ausführungsform sind die Verstärkungsschlaufe und wenigstens eine andere Schlaufe in Längsrichtung des Gittergeflechts hintereinander angeordnet und bilden eine Mehrfachschlaufe. Die Mehrfachschlaufe kann also mehrere Schlaufen umfassen, die in Längsrichtung des Gittergeflechts hintereinander angeordnet sind, wobei wenigstens eine Schlaufe als Verstärkungsschlaufe ausgebildet ist. Durch die Mehrfachschlaufen kann die Verankerung der medizinischen Vorrichtung in einem Körperhohlorgan weiter verbessert werden.

Es ist möglich, dass mehrere Mehrfachschlaufen vorgesehen sind, die in Umfangsrichtung des Gittergeflechts nebeneinander angeordnet sind. Im Allgemeinen sind die Schlaufen am Längsende des Gittergeflechts, die nicht derselben Mehrfachschlaufe angehören, nebeneinander angeordnet.

In einer besonders bevorzugten Ausgestaltung der erfindungsgemäßen medizinischen Vorrichtung bildet die Verstärkungsschlaufe eine äußere Schlaufe der Mehrfachschlaufe. Alternativ kann die Verstärkungsschlaufe auch eine innere Schlaufe der Mehrfachschlaufe bilden. Insgesamt ist vorgesehen, dass die Mehrfachschlaufe eine äußere Schlaufe aufweist, die das Längsende des Gittergeflechts begrenzt und durch eine Verstärkungsschlaufe gebildet ist. Die Mehrfachschlaufe kann weitere Schlaufen umfassen, die gegenüber der äußeren Schlaufe zurückversetzt bzw. in Richtung zur Geflechtmitte hinter der äußeren Schlaufe angeordnet sind und als innere Schlaufen bezeichnet werden. Die äußere Schlaufe und die inneren Schlaufen sind in Längsrichtung des Gittergeflechts hintereinander angeordnet.

Die Verstärkungsschlaufe und die andere Schlaufe der Mehrfachschlaufe können in einem expandierten Zustand des Gittergeflechts zumindest abschnittsweise deckungsgleich übereinander und in einem komprimierten Zustand des Gittergeflechts beabstandet voneinander angeordnet sein. Es ist bevorzugt, wenn die Verstärkungsschlaufe und die andere Schlaufe, die gemeinsam einer Mehrfachschlaufe angehören, derart angeordnet sind, dass in einem komprimierten Zustand zwischen der Verstärkungsschlaufe und der anderen Schlaufe ein Abstand besteht. Dadurch wird der Querschnittsdurchmesser des Längsendes des Gittergeflechts im komprimierten Zustand reduziert. Gleichzeitig wird die Radialkraft, die im komprimierten Zustand des Gittergeflechts beispielsweise auf eine Katheterinnenwand wirkt, verringert, so dass die Zuführung der medizinischen Vorrichtung in ein Körperhohlorgan erleichtert ist.

Ferner ist vorgesehen, dass die Verstärkungsschlaufe die andere Schlaufe der Mehrfachschlaufe in einem expandierten Zustand des Gittergeflechts derart überlappt, dass die Verstärkungsschlaufe und die andere Schlaufe im Wesentlichen deckungsgleich aufeinanderliegend angeordnet sind. In diesem Fall unterstützt die andere Schlaufe die von der Verstärkungsschlaufe bewirkte Radialkraft, so dass sich im expandierten Zustand des Gittergeflechts am Längsende eine erhöhte Radialkraft einstellt, die zu einer verbesserten Verankerung des Gittergeflechts in einem Körperhohlorgan beiträgt. Vorzugsweise ist die Anordnung der Verstärkungsschlaufe und der anderen Schlaufe einer Mehrfachschlaufe daher derart ausgelegt, dass sich die Verstärkungsschlaufe und die andere Schlaufe in einem expandierten Zustand des Gittergeflechts überlappen, der im Wesentlichen dem Implantationszustand entspricht. Die Überlappung erfolgt also vorteilhaft bei einem Querschnittsdurchmesser des Gittergeflechts bzw. konkret des Längsendes des Gittergeflechts, der sich am Behandlungsort in dem entsprechenden Körperhohlorgan einstellt.

Die Erfindung wird im Folgenden anhand von Ausführungsbeispielen unter Bezugnahme auf die beigefügten, schematischen Zeichnungen näher erläutert. Darin zeigen
- Fig. 1: eine Abwicklung eines Gittergeflechts einer nicht anspruchsgemäßen medizinischen Vorrichtung im expandierten Zustand, wobei ca. 50% der Schlaufen des Gittergeflechts als Verstärkungsschlaufen ausgebildet sind;
- Fig. 2 bis 3: jeweils eine Abwicklung eines Gittergeflechts einer beanspruchten erfindungsgemäßen medizinischen Vorrichtung nach einem bevorzugten Ausführungsbeispiel im expandierten Zustand, wobei ca. 33% (Fig. 2) bzw. ca. 17% (Fig. 3) der Schlaufen des Gittergeflechts als Verstärkungsschlaufen ausgebildet sind;
- Fig. 4: eine Abwicklung eines Gittergeflechts einer erfindungsgemäßen medizinischen Vorrichtung nach einem bevorzugten Ausführungsbeispiel im expandierten Zustand, wobei sich die Verstärkungsschlaufe mit einer anderen Schlaufe einer Mehrfachschlaufe abschnittsweise überdeckt;
- Fig. 5, 6: jeweils eine Abwicklung eines Gittergeflechts einer erfindungsgemäßen medizinischen Vorrichtung nach einem weiteren bevorzugten Ausführungsbeispiel im expandierten Zustand, wobei Mehrfachschlaufen vorgesehen sind, deren äußere Schlaufe durch eine Verstärkungsschlaufe gebildet ist; und
- Fig. 7: eine Abwicklung eines Gittergeflechts der erfindungsgemäßen medizinischen Vorrichtung nach einem weiteren bevorzugten Ausführungsbeispiel im expandierten Zustand, wobei Mehrfachschlaufen mit einer inneren Schlaufe vorgesehen sind, die als Verstärkungsschlaufe ausgebildet ist.

Die erfindungsgemäße medizinische Vorrichtung ist vorzugsweise als Stent, Stentgraft, Flow Diverter, Thrombektomie-Device oder Thrombenfilter einsetzbar bzw. ausgebildet. Im Allgemeinen kann die medizinische Vorrichtung ein medizinisches Implantat oder ein medizinisches Instrument bilden, das über ein Zuführsystem, insbesondere einen Katheter, minimalinvasiv in den menschlichen Körper einführbar ist.

Die Vorrichtung umfasst ein expandierbares Gittergeflecht 10, das mehrere miteinander verflochtene Drähte 11, 12 aufweist. Die Drähte 11, 12 überkreuzen sich und bilden Kreuzungspunkte. Ferner begrenzen die Drähte 11, 12 sowie die Kreuzungspunkte Maschen 16 des Gittergeflechts 10. Vorzugsweise ist das Gittergeflecht 10 in einer Wandungsebene der medizinischen Vorrichtung angeordnet, die sich rotationssymmetrisch um eine Längsachse der medizinischen Vorrichtung erstreckt. Die Wandungsebene, in der das Gittergeflecht 10 angeordnet ist, kann kreiszylinderartig geformt sein. Die Drähte 11, 12 verlaufen innerhalb des Gittergeflechts 10 schraubenförmig um die Längsachse des Gittergeflechts 10. Dabei können die Drähte 11, 12 zumindest abschnittsweise gegenläufig schraubenförmig um die Längsachse verlaufen, so dass sich die Drähte 11, 12 regelmäßig kreuzen.

An wenigstens einem Längsende 15 des Gittergeflechts 10 bilden die Drähte 11, 12 Schlaufen. Die Drähte 11, 12 sind also am Längsende 15 des Gittergeflechts 10 umgelenkt und in das Gittergeflecht 10 zurückgeführt, wodurch geschlossene Schlaufen 13, 14 gebildet sind. Vorzugsweise sind die Schlaufen 13, 14 jeweils durch einen Draht 11, 12 gebildet, der einstückig ausgebildet ist.

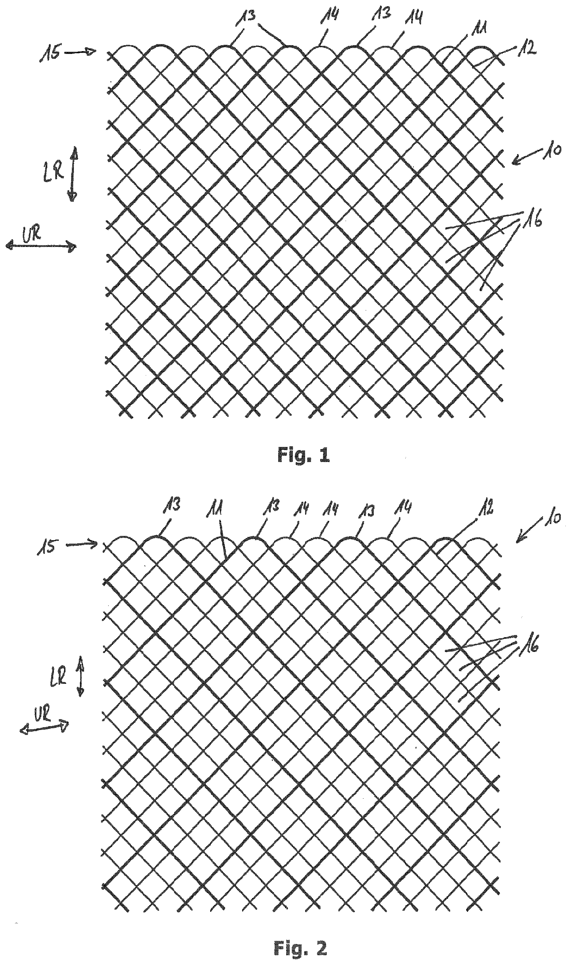

Wie in Fig. 1 dargestellt, weist das Gittergeflecht 10 mehrere Schlaufen 13, 14 auf, die das Längsende 15 des Gittergeflechts 10 begrenzen. Die Schlaufen 13, 14 sind durch Drähte 11, 12 gebildet, die sich hinsichtlich ihres Drahtquerschnitts bzw.

Querschnittsdurchmessers unterscheiden. Insbesondere ist ein Teil der Schlaufen 13, 14 als Verstärkungsschlaufen 13 ausgebildet, wobei die Verstärkungsschlaufen 13 jeweils durch einen Draht 11 gebildet sind, der einen größeren Drahtquerschnitt aufweist als die Drähte 12 der anderen Schlaufen 14. Insbesondere weist der Draht 11 der Verstärkungsschlaufe 13 zumindest im Bereich der Verstärkungsschlaufe 13 einen größeren Drahtquerschnitt als die Drähte 12 der anderen Schlaufen 14 auf. Bei den in den Figuren dargestellten Ausführungsbeispielen ist der Drahtquerschnitt innerhalb eines Drahts 11, 12 konstant.

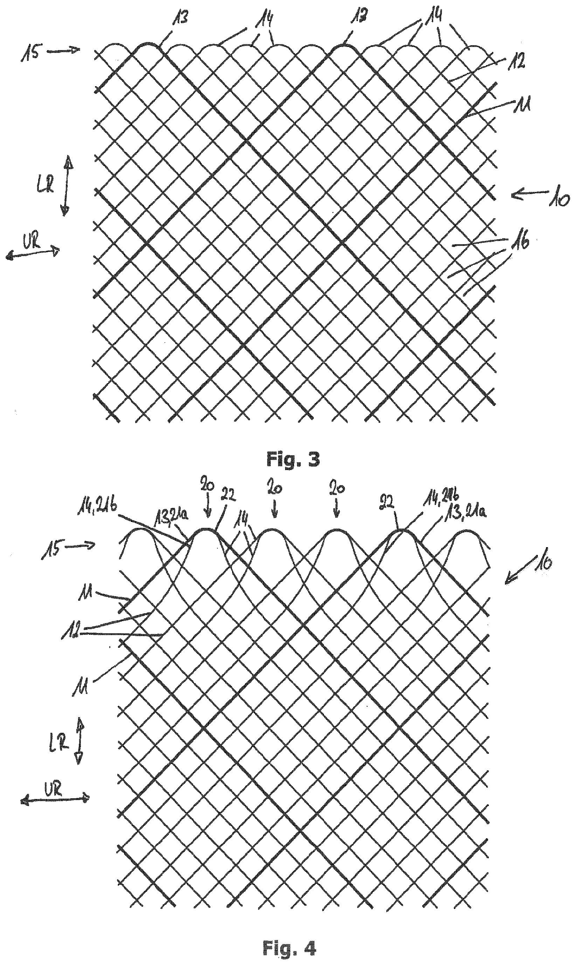

Die Verstärkungsschlaufen 13 sind musterartig über das Längsende 15 verteilt angeordnet. Bei dem nicht anspruchsgemäßen Ausführungsbeispiel gemäß Fig. 1 sind beispielsweise 50% der Schlaufen 13, 14 des Längsendes 15 des Gittergeflechts 10 als Verstärkungsschlaufen 13 ausgebildet. Mit anderen Worten, ist jede zweite Schlaufe 13, 14 als Verstärkungsschlaufe 13 ausgebildet. Bei dem anspruchsgemäßen Ausführungsbeispiel gemäß Fig. 2 ist hingegen jede dritte Schlaufe 13, 14 als Verstärkungsschlaufe 13 ausgebildet. Dies entspricht einem prozentualen Anteil von etwa 33% der Schlaufen 13, 14 eines Längsendes 15. Fig. 3 zeigt ein Ausführungsbeispiel der erfindungsgemäßen medizinischen Vorrichtung, wobei am Längsendes 15 des Gittergeflechts 10 etwa 17% der Schlaufen 13, 14 als Verstärkungsschlaufen 13 ausgebildet sind. Konkret ist gemäß Fig. 3 jede sechste Schlaufe als Verstärkungsschlaufe ausgebildet.

Bei den Ausführungsbeispielen gemäß Fig. 1 bis 3 weist das Gittergeflecht 10 am Längsende 15 jeweils einfache Schlaufen 13, 14 auf. Die Schlaufen 13, 14 sind dabei in Umfangsrichtung des Gittergeflechts 10 benachbart bzw. nebeneinander angeordnet.

In diesem Zusammenhang wird darauf hingewiesen, dass die Umfangsrichtung in den beigefügten Figuren horizontal in der Zeichnungsebene orientiert und durch einen mit UR bezeichneten Doppelpfeil dargestellt ist. Die Längsrichtung des Gittergeflechts 10 verläuft in den beigefügten Zeichnungen in der Zeichnungsebene vertikal und ist mit einem mit LR bezeichneten Doppelpfeil gekennzeichnet.

Das Gittergeflecht 10 kann an einem Längsende 15 auch Mehrfachschlaufen 20 aufweisen, wie beispielsweise in Fig. 4 dargestellt. Im Unterschied zu einfachen Schlaufen 13, 14, wie in den Fig. 1 bis 3 gezeigt, sind die Mehrfachschlaufen 20 durch wenigstens zwei Schlaufen 13, 14 gebildet, die in Längsrichtung des Gittergeflechts 10 hintereinander angeordnet sind. Bei dem Ausführungsbeispiel gemäß Fig. 4 weist das Längsende 15 mehrere Mehrfachschlaufen 20 auf, die durch jeweils zwei Einzelschlaufen 13, 14 gebildet sind. Fig. 4 zeigt einen expandierten Zustand des Gittergeflechts 10, in dem sich die Schlaufen 13, 14 zumindest abschnittsweise deckungsgleich überlappen. Insbesondere ist ein Überlappungsbereich 22 vorgesehen, in dem jeweils ein Abschnitt einer äußeren Schlaufe 21a einen Abschnitt einer inneren Schlaufe 21b überlappt. Die von den in radialer Richtung bezogen auf die Längsachse des Gittergeflechts 10 aufeinanderliegenden Schlaufen 13, 14 bewirkte Radialkraft wird dabei im Überlappungsbereich 22 summiert.

Das Gittergeflecht 10 gemäß Fig. 4 weist Verstärkungsschlaufen 13 auf. Insbesondere umfasst jede dritte Mehrfachschlaufe 20 eine Verstärkungsschlaufe 13, die durch einen Draht 11 gebildet ist, dessen Drahtquerschnitt größer als der Drahtquerschnitt eines Drahtes 12 ist, der die andere Schlaufe 14 derselben Mehrfachschlaufe 20 bildet. Die Verstärkungsschlaufe 13 bildet dabei eine äußere Schlaufe 21a der Mehrfachschlaufe 20. Insgesamt weist die Mehrfachschlaufe 20 eine äußere Schlaufe 21a und eine innere Schlaufe 21b auf. Die innere Schlaufe 21b ist dem Inneren des Gittergeflechts 10 näher als die äußere Schlaufe 21a, die das Längsende 15 des Gittergeflechts begrenzt. Die äußere Schlaufe 21a ist vorzugsweise durch die Verstärkungsschlaufe 13 gebildet. Im Übrigen entspricht die Gestaltung der Mehrfachschlaufen 20 gemäß Fig. 4 der Ausgestaltung eines Gittergeflechts 10, wie es beispielsweise in der auf die Anmelderin zurückgehenden und am gleichen Tag eingereichten deutschen Patentanmeldung "Medizinische Vorrichtung mit expandierbarem Gittergeflecht", insbesondere auf Seiten 7 bis 18, beschrieben ist.

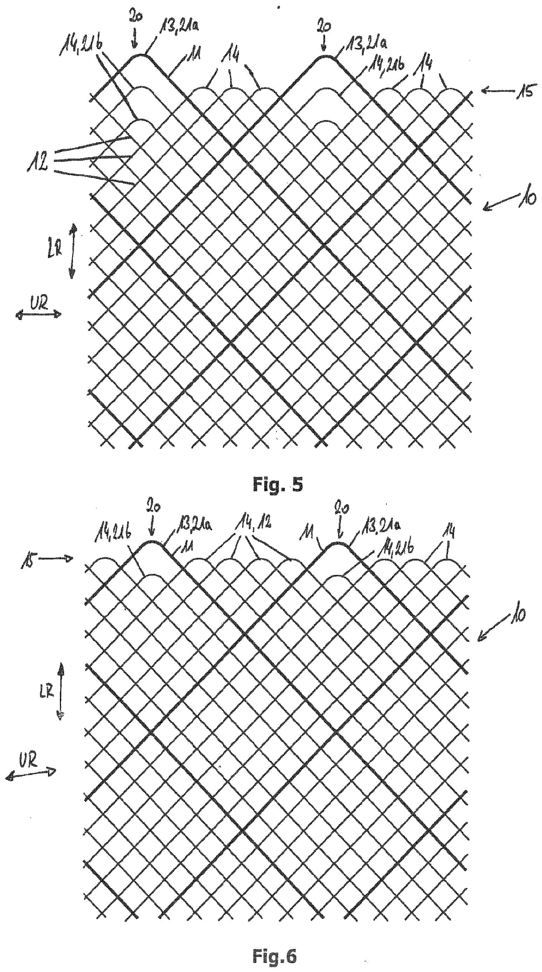

In den Ausführungsbeispielen gemäß Fig. 1 bis 4 sind die Schlaufen 13, 14 bzw. die Mehrfachschlaufen 20 entlang einer gemeinsamen Umfangslinie des Gittergeflechts 10 angeordnet, so dass sich insgesamt ein geradlinig begrenztes Längsende 15 bildet. Alternativ kann vorgesehen sein, dass einzelne Schlaufen 13, 14 über das Längsende 15 bzw. über die anderen Schlaufen 13, 14 vorstehen. In Fig. 5 ist ein Ausführungsbeispiel dargestellt, wobei Verstärkungsschlaufen vorgesehen sind, die über die anderen Schlaufen 14 des Längsendes 15 vorstehen. Insbesondere ist vorgesehen, dass die Verstärkungsschlaufen 13 jeweils einer Mehrfachschlaufe 20 zugeordnet sind bzw. mit weiteren Schlaufen 14 eine Mehrfachschlaufe 20 bilden. Die Mehrfachschlaufe 20 weist eine äußere Schlaufe 21a und zwei innere Schlaufen 21b auf, wobei die äußere Schlaufe 21a als Verstärkungsschlaufe 13 ausgebildet ist. Eine andere Anzahl von inneren Schlaufen 21b ist möglich. Im Allgemeinen kann die Mehrfachschlaufe 20 mehrere einzelne Schlaufen 13, 14 umfassen, wobei eine oder mehrere einzelne Schlaufen 13, 14 als Verstärkungsschlaufen 13 ausgebildet sind. Insbesondere können auch alle einzelnen Schlaufen 13, 14 einer Mehrfachschlaufe 20 als Verstärkungsschlaufe ausgebildet sein.

Zwischen jeweils zwei Mehrfachschlaufen 20 bzw. Verstärkungsschlaufen 13 sind in Umfangsrichtung des Gittergeflechts 10 drei Einzelschlaufen 14 angeordnet. Mit anderen Worten ist an dem Längsende 15 des Gittergeflechts jede vierte Schlaufe als Mehrfachschlaufe 20 bzw. als Verstärkungsschlaufe 13 ausgebildet. Andere Muster sind möglich.

Einen ähnlichen Aufbau weist das Gittergeflecht gemäß dem Ausführungsbeispiel nach Fig. 6 auf, wobei jede fünfte Schlaufe 13, 14 des Längsendes 15 des Gittergeflechts 10 als Mehrfachschlaufe 20 bzw. als Verstärkungsschlaufe 13 ausgebildet ist. Die Mehrfachschlaufen 20 des Gittergeflechts 10 nach Fig. 6 weisen jeweils eine äußere Schlaufe 21a und eine innere Schlaufe 21b auf, wobei die äußere Schlaufe 21a als Verstärkungsschlaufe 13 ausgebildet ist. Die Verstärkungsschlaufe 13 steht über die anderen Schlaufen 14, die als Einzelschlaufen ausgebildet sind, vor.

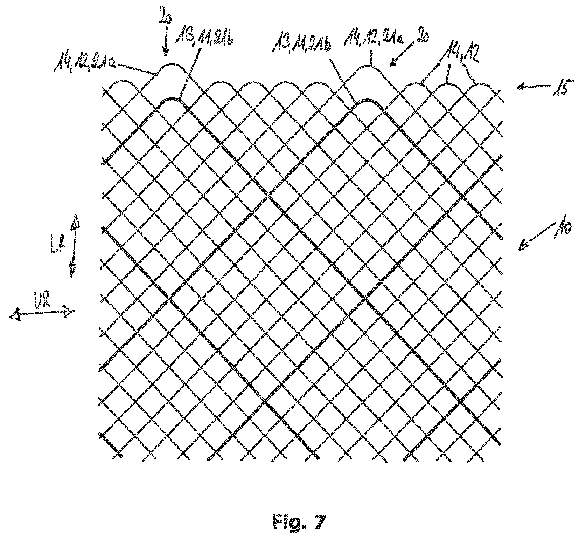

Das Ausführungsbeispiel gemäß Fig. 7 entspricht im Wesentlichen dem Ausführungsbeispiel gemäß Fig. 6, mit dem Unterschied, dass die innere Schlaufe 21b der Mehrfachschlaufe 20 als Verstärkungsschlaufe 13 ausgebildet ist. Dabei ist die Verstärkungsschlaufe 13 gegenüber den anderen Schlaufen 14, die als Einzelschlaufen ausgebildet sind, zurückgesetzt.

Im Allgemeinen wird darauf hingewiesen, dass die Darstellungen gemäß Fig. 1 bis 7 jeweils einen expandierten Zustand, insbesondere den vollständig expandierten Zustand des Gittergeflechts 10 zeigen.

Im Folgenden werden bevorzugte Varianten eines Gittergeflechts 10 mit Mehrfachschlaufen 20 beschrieben:
Vorzugsweise umfasst das Gittergeflecht 10 an einem Längsende 15 zwölf Doppelschlaufen, also Mehrfachschlaufen 20, die aus zwei Schlaufen 13, 14 gebildet sind. Zwei der zwölf Doppelschlaufen weisen jeweils eine Verstärkungsschlaufe 13 auf. Die übrigen zehn Doppelschlaufen sind hingegen aus anderen Schlaufen 14 gebildet, deren Drähte 12 jeweils denselben Querschnittsdurchmesser aufweisen. In den beiden Doppelschlaufen, die eine Verstärkungsschlaufe 13 aufweisen, ist vorzugsweise die äußere Schlaufe als Verstärkungsschlaufe 13 ausgebildet. Dabei weist der Draht 11, der die Verstärkungsschlaufe 13 bildet, vorzugsweise einen Querschnittsdurchmesser von 85 µm auf. Die Drähte 12 der anderen Schlaufen 14 umfassen vorzugsweise einen Querschnittsdurchmesser von 35 µm.

Mit anderen Worten weist das Längsende 15 des Gittergeflechts 10 vorzugsweise 12 Doppelschlaufen auf, die jeweils eine innere Schlaufe 21b aufweisen, deren Draht 12 einen Querschnittsdurchmesser von 35 µm aufweist. Zwei der 12 Doppelschlaufen weisen jeweils eine äußere Schlaufe 21a auf, deren Draht einen Querschnittsdurchmesser von 85 µm umfasst. Der Draht der jeweils äußeren Schlaufe 21a der übrigen zehn Doppelschlaufen weist hingegen denselben Querschnittsdurchmesser wie der Draht der inneren Schlaufen 21b, also 35 µm, auf.

Insgesamt weist das Längsende 15 also 24 einzelne Schlaufen 13, 14 auf, wobei zwei der einzelnen Schlaufen 13, 14 als Verstärkungsschlaufen 13 ausgebildet sind.

Grundsätzlich ist es vorteilhaft, eine geringe Anzahl von Verstärkungsschlaufen 13 vorzusehen, wobei jedoch der Querschnittsdurchmesser der Drähte, die die Verstärkungsschlaufen 13 bilden, relativ groß gewählt ist. Auf diese Weise lässt sich die Radialkraft steigern.

Es ist bevorzugt, wenn mindestens zwei, insbesondere mindestens drei, insbesondere mindestens vier, insbesondere mindestens sechs, Mehrfachschlaufen 20 von insgesamt zwölf Mehrfachschlaufen 20, die ein Längsende 15 eines Gittergeflechts 10 bilden, jeweils eine Verstärkungsschlaufe 13 aufweisen, oder generell aus Schlaufen 13, 14 gebildet sind, deren Drähte 11, 12 unterschiedliche Querschnittsdurchmesser aufweisen. Dadurch wird ein besonders homogenes Gittergeflecht 10 bezogen auf die Verteilung der Radialkraft bereitgestellt. Generell ist bevorzugt, dass das Verhältnis zwischen der Anzahl der Mehrfachschlaufen mit einer oder mehreren Verstärkungsschlaufen zur Gesamtanzahl der Mehrfachschlaufen mindestens 1/12, insbesondere mindestens 1/6, insbesondere mindestens 1/4, insbesondere mindestens 1/3, insbesondere mindestens 1/2 beträgt.

Alternativ kann vorgesehen sein, dass höchstens sechs, insbesondere höchstens vier, insbesondere höchstens drei, insbesondere höchstens zwei Mehrfachschlaufen 20 einer Gesamtzahl von zwölf Mehrfachschlaufen 20 an einem Längsende 15 des Gittergeflechts 10 aus Drähten mit unterschiedlichem Querschnittsdurchmesser gebildet ist. Mit anderen Worten weisen höchstens sechs, insbesondere höchstens vier, insbesondere höchstens drei, insbesondere höchstens zwei, Mehrfachschlaufen 20 von insgesamt zwölf Mehrfachschlaufen 20 eine oder mehrere Verstärkungsschlaufen 13 auf. Durch die Begrenzung der Anzahl von Verstärkungsschlaufen 13 wird ermöglicht, den Querschnittsdurchmesser des Drahts, der die Verstärkungsschlaufe 13 bildet, zu erhöhen, ohne den Gesamtdurchmesser des Gittergeflechts bzw. des Längsendes 15, insbesondere im komprimierten Zustand, negativ zu beeinflussen. Generell ist bevorzugt, dass das Verhältnis zwischen der Anzahl der Mehrfachschlaufen mit einer oder mehreren Verstärkungsschlaufen zur Gesamtanzahl der Mehrfachschlaufen höchstens 1/2, insbesondere höchstens 1/3, insbesondere höchstens 1/4, insbesondere höchstens 1/6, insbesondere höchstens 1/12 beträgt.

Es ist auch möglich, dass die Mehrfachschlaufen 20 mehr als eine Verstärkungsschlaufe 13 aufweisen. Insbesondere ist vorgesehen, dass das Gittergeflecht 10 insbesondere sechs, insbesondere höchstens vier, insbesondere höchstens drei, insbesondere höchstens zwei, Mehrfachschlaufen 20 aufweist, die jeweils wenigstens eine Verstärkungsschlaufe 13, insbesondere ausschließlich eine einzige Verstärkungsschlaufe 13, aufweisen.

Das Verhältnis der Anzahl von Verstärkungsschlaufen zu der Gesamtanzahl der Schlaufen pro Mehrfachschlaufe kann höchstens 1/2, insbesondere höchstens 1/4, insbesondere höchstens 1/6 betragen. Die Untergrenze beträgt eine einzige Verstärkungsschlaufe.

Das Verhältnis der Anzahl von Verstärkungsschlaufen 13 zu der Gesamtanzahl der Schlaufen beträgt höchstens 34%-, insbesondere höchstens 1:4, insbesondere höchstens 1:6, insbesondere höchstens 1:8, insbesondere höchstens 1:10, insbesondere höchstens 1:12, insbesondere höchstens 1:16, insbesondere höchstens 1:18, insbesondere höchstens 1:24. Beispielsweise, aber außerhalb des Schutzumfangs der Ansprüche können an dem Längsende 15 des Gittergeflechts 10 ausschließlich Doppelschlaufen vorgesehen sein, die jeweils eine einzige Verstärkungsschlaufe 13 umfassen, um ein Verhältnis zwischen der Anzahl der Verstärkungsschlaufen 13 und Gesamtanzahl der Schlaufen von 1:2 einzustellen. Im Allgemeinen ist es bevorzugt, wenn die Verstärkungsschlaufen 13 jeweils die äußeren Schlaufen 21a einer Mehrfachschlaufe 20 bilden.

Die Verstärkungsschlaufen 13 des Gittergeflechts 10 sind jeweils durch Drähte 11 gebildet, die denselben Querschnittsdurchmesser aufweisen. Die Drähte 12 der weiteren Schlaufen 14 weisen ebenfalls einen einheitlichen Querschnittsdurchmesser auf.

Insbesondere ist vorgesehen, dass die Drähte 14 der anderen Schlaufen 14, die gemeinsam mit wenigstens einer Verstärkungsschlaufe 13 eine Doppel- oder Mehrfachschlaufe 20 bilden, denselben Querschnittsdurchmesser aufweisen wie die Drähte 12 der anderen Schlaufen 14, die entweder als Einzelschlaufen am Längsende 15 vorliegen oder eine Mehrfachschlaufe 20 bilden, die ausschließlich aus anderen Schlaufen 14, also ohne eine Verstärkungsschlaufe 13, gebildet ist.

Die Drähte 11, die die Verstärkungsschlaufen 13 bilden, weisen zumindest im Bereich der Verstärkungsschlaufe 13 vorzugsweise einen Querschnittsdurchmesser von wenigstens 50 µm, insbesondere wenigstens 60 µm, insbesondere wenigstens 70 µm, insbesondere wenigstens 80 µm, insbesondere wenigstens 85 µm, insbesondere wenigstens 100 µm, insbesondere wenigstens 150 µm, auf. Die Drähte 12, die die anderen Schlaufen 14 bilden, weisen hingegen vorzugsweise einen Querschnittsdurchmesser auf, der höchstens 70 µm, insbesondere höchstens 50 µm, insbesondere höchstens 40 µm, insbesondere höchstens 35 µm, insbesondere höchstens 30 µm, insbesondere höchstens 25 µm, insbesondere höchstens 20 µm, umfasst. Dabei ist vorteilhaft ein Verhältnis zwischen dem Querschnittsdurchmesser der Drähte, die die anderen Schlaufen bilden, und dem Querschnittsdurchmesser der Drähte 11, die die Verstärkungsschlaufen 13 bilden, von höchstens 0,8, insbesondere höchstens 0,7, insbesondere höchstens 0,6, insbesondere höchstens 0,5, insbesondere höchstens 0,4, insbesondere höchstens 0,3, insbesondere höchstens 0,2, insbesondere höchstens 0,1, vorgesehen.

Die Schlaufen, insbesondere die Mehrfachschlaufen 20, die Verstärkungsschlaufen 13, die anderen Schlaufen 14, die äußeren Schlaufe 21a, die inneren Schlaufe 21b sind an wenigstens einem axialen Ende bzw. Längsende 15 des Gittergeflechts 10, insbesondere an einem einzigen oder an beiden Enden des Gittergeflechts 10 angeordnet.

### Bezugszeichenliste

- 10: Gittergeflecht
- 11: Draht der Verstärkungsschlaufe 13
- 12: Draht der anderen Schlaufe 14
- 13: Verstärkungsschlaufe
- 14: andere Schlaufe
- 15: Längsende
- 16: Masche
- 20: Mehrfachschlaufe
- 21a: äußere Schlaufe
- 21b: innere Schlaufe
- 22: Überlappungsbereich
- LR: Längsrichtung
- UR: Umfangsrichtung

## Patentansprüche

1. Medizinische Vorrichtung mit einem expandierbaren Gittergeflecht (10) aus sich überkreuzenden Drähten (11, 12), die an wenigstens einem Längsende (15) des Gittergeflechts (10) Schlaufen (13, 14) bilden, wobei wenigstens eine Schlaufe (13, 14) als Verstärkungsschlaufe (13) ausgebildet ist, die durch einen Draht (11) gebildet ist, der im Bereich der Verstärkungsschlaufe (13) einen größeren Querschnittsdurchmesser als die Drähte (12) aufweist, die die anderen Schlaufen (14) bilden, wobei mehrere Verstärkungsschlaufen (13) vorgesehen sind,
**dadurch gekennzeichnet, dass**
die Verstärkungsschlaufen (13) musterartig über das Längsende (15) verteilt angeordnet sind, wobei höchstens 34% der Schlaufen (13, 14) als Verstärkungsschlaufen (13) ausgebildet sind.

2. Medizinische Vorrichtung nach Anspruch 1,
**dadurch gekennzeichnet, dass**
höchstens 17%, der Schlaufen (13, 14) als Verstärkungsschlaufen (13) ausgebildet sind.

3. Medizinische Vorrichtung nach Anspruch 1 oder 2,
**dadurch gekennzeichnet, dass**
die Verstärkungsschlaufe (13) in Längsrichtung des Gittergeflechts (10) über die anderen Schlaufen (14) vorsteht oder bezogen auf die anderen Schlaufen (14) zurückgesetzt ist.

4. Medizinische Vorrichtung nach einem der Ansprüche 1 bis 3,
**dadurch gekennzeichnet, dass**
die Verstärkungsschlaufe (13) und wenigstens eine andere Schlaufe (14) in Längsrichtung des Gittergeflechts (10) hintereinander angeordnet sind und eine Mehrfachschlaufe (20) bilden.

5. Medizinische Vorrichtung nach Anspruch 4,
**dadurch gekennzeichnet, dass**
die Verstärkungsschlaufe (13) eine äußere Schlaufe (21a) oder eine innere Schlaufe (21b) der Mehrfachschlaufe (20) bildet.

6. Medizinische Vorrichtung nach Anspruch 4 oder 5,
**dadurch gekennzeichnet, dass**
die Verstärkungsschlaufe (13) und die andere Schlaufe (14) der Mehrfachschlaufe (20) in einem expandierten Zustand des Gittergeflechts (10) zumindest abschnittsweise deckungsgleich übereinander und in einem komprimierten Zustand des Gittergeflechts (10) beabstandet voneinander angeordnet sind.

7. Medizinische Vorrichtung nach einem der Ansprüche 4 bis 6,
**dadurch gekennzeichnet, dass**
das Verhältnis der Mehrfachschlaufen mit mindestens einer Verstärkungsschlaufe (13) zu der Gesamtzahl der Mehrfachschlaufen höchstens 1/2, insbesondere höchstens 1/3, insbesondere höchstens 1/4, insbesondere höchstens 1/6, insbesondere höchstens 1/12, beträgt.

## Claims

1. A medical device comprising an expandable lattice mesh (10) consisting of intersecting wires (11, 12) which form loops (13, 14) on at least one longitudinal end (15) of the lattice mesh (10), wherein at least one loop (13, 14) is configured as a reinforcement loop (13) which is formed by a wire (11) which, in the region of the reinforcement loop (13), has a larger cross-sectional diameter than the wires (12) which form the other loops (14),
wherein several reinforcement loops (13) are provided,
**characterized in that** the reinforcement loops (13) are arranged distributed in the manner of a pattern over the longitudinal end (15), wherein at most 34 % of the loops (13, 14) are configured as reinforcement loops (13).

2. The medical device according to claim 1,
**characterized in that**
at most 17 % of the loops (13, 14) are configured as reinforcement loops (13).

3. The medical device according to claim 1 or 2,
**characterized in that**
the reinforcement loop (13) projects over the other loops (14) in longitudinal direction of the lattice mesh (10) or is recessed with respect to the other loops (14).

4. The medical device according to one of claims 1 to 3,
**characterized in that**
the reinforcement loop (13) and at least one other loop (14) are arranged one behind the other in longitudinal direction of the lattice mesh (10) and form a multiple loop (20).

5. The medical device according to claim 4,
**characterized in that**
the reinforcement loop (13) forms an outer loop (21a) or an inner loop (21b) of the multiple loop (20).

6. The medical device according to claim 4 or 5,
**characterized in that**
in an expanded state of the lattice mesh (10), the reinforcement loop (13) and the other loop (14) of the multiple loop (20) are arranged at least partially congruently over one another and, in a compressed state of the lattice mesh (10), are arranged spaced apart from one another.

7. The medical device according to one of claims 4 to 6,
**characterized in that**
the ratio of the multiple loops with at least one reinforcement loop (13) to the total number of the multiple loops is at most 1/2, in particular at most 1/3, in particular at most 1/4, in particular at most 1/6, in particular at most 1/12.

## Revendications

1. Dispositif médical avec un treillis expansible (10) constitué de fils (11, 12) entrecroisés qui forment des boucles (13, 14) à au moins une extrémité longitudinale (15) du treillis (10), dans lequel au moins une boucle (13, 14) est réalisée en tant que boucle de renforcement (13) qui est formée par un fil (11) présentant, dans la zone de la boucle de renforcement (13), un diamètre transversal supérieur à celui des fils (12) qui forment les autres boucles (14),
dans lequel on prévoit plusieurs boucles de renforcement (13),
**caractérisé en ce que** les boucles de renforcement (13) sont disposées de façon répartie à la manière d'un motif sur l'extrémité longitudinale (15), dans lequel un maximum de 34 % des boucles (13, 14) sont réalisées en tant que boucles de renforcement (13).

2. Dispositif médical selon la revendication 1,
**caractérisé en ce**
**qu'**un maximum de 17 % des boucles (13, 14) sont réalisées en tant que boucles de renforcement (13).

3. Dispositif médical selon la revendication 1 ou 2,
**caractérisé en ce que**
la boucle de renforcement (13) est en saillie en direction longitudinale du treillis (10) par rapport aux autres boucles (14) ou est en retrait compte tenu des autres boucles (14).

4. Dispositif médical selon l'une des revendications 1 à 3,
**caractérisé en ce que**
la boucle de renforcement (13) et au moins une autre boucle (14) sont disposées l'une derrière l'autre en direction longitudinale du treillis (10) et forment une boucle multiple (20).

5. Dispositif médical selon la revendication 4,
**caractérisé en ce que**
la boucle de renforcement (13) forme une boucle extérieure (21a) ou une boucle intérieure (21b) de la boucle multiple (20).

6. Dispositif médical selon la revendication 4 ou 5,
**caractérisé en ce que**,
dans un état d'expansion du treillis (10), la boucle de renforcement (13) et l'autre boucle (14) de la boucle multiple (20) sont disposées au moins partiellement l'une au-dessus de l'autre de façon coïncidente, et sont disposées à distance l'une de l'autre dans un état comprimé du treillis (10).

7. Dispositif médical selon l'une des revendications 4 à 6,
**caractérisé en ce que**
le rapport entre les boucles multiples avec au moins une boucle de renforcement (13) et le nombre total des boucles multiples est d'un maximum de 1/2, en particulier d'un maximum de 1/3, en particulier d'un maximum de 1/4, en particulier d'un maximum de 1/6, en particulier d'un maximum de 1/12.
